# EUROPÄISCHE PATENTSCHRIFT

(11) **EP 3 650 797 B1**
(45) Veröffentlichungstag und Bekanntmachung des Hinweises auf die Patenterteilung: **11.09.2024**
(21) Anmeldenummer: 19207150.4
(22) Anmeldetag: 05.11.2019
(51) Int. Cl.: F28D 9/00, F28F 3/08, F28F 3/12, F28F 13/12, A61M 1/36

(54) **VORRICHTUNG ZUR EXTRAKORPORALEN TEMPERIERUNG VON PATIENTEN MIT EINEM TRENNBAREN SEKUNDÄRKÖRPER**
DEVICE FOR EXTRACORPOREAL TEMPERING OF PATIENTS WITH A SEPARABLE SECONDARY BODY
DISPOSITIF DE THERMORÉGULATION EXTRACORPORALE DE PATIENTS COMPRENANT UN SECOND CORPS SÉPARABLE

(30) Priorität: 09.11.2018 DE 102018128102
(43) Veröffentlichungstag der Anmeldung: 13.05.2020
(73) Patentinhaber: Lauda Dr. R. Wobser GmbH & Co. KG, 97922 Lauda-Königshofen (DE)
(72) Erfinder: Braun, Kristofer, 97980 Bad Mergentheim (DE)
(74) Vertreter: Zimmermann & Partner Patentanwälte mbB

(56) Entgegenhaltungen:
- US-A- 4 156 459
- US-A1- 2009 235 686
- US-A1- 2009 255 655
- US-A1- 2015 230 975
- US-A1- 2016 082 175
- US-A1- 2018 207 027

## Beschreibung

### Gebiet der Erfindung

Die Erfindung betrifft eine Vorrichtung zur extrakorporalen Temperierung von Patienten.

### Stand der Technik

Zur extrakorporalen Temperierung von Patienten sind Verfahren und Vorrichtungen bekannt, welche durch Verwendung von Wärmetauschern gekennzeichnet sind. Bei den Wärmetauschern wird in vielen Fällen Wasser als Medium zum Transport von Wärme oder Kälte verwendet. Die US 2015/0230975 A1 betrifft ein Patienten-Wärmetauschsystem mit zwei Fluidkreisläufen. Die US 4 156 459 A betrifft einen Plattenverdampfer zum Verdampfen einer Flüssigkeit mittels eines Heizmediums. Innere Platten des Plattenverdampfers weisen Öffnungen auf.

Allerdings haben bisher bekannte Lösungen aus dem Stand der Technik Restriktionen in Bezug auf die übertragbare Wärmeleistung oder sind nur aufwendig zu reinigen.

### Offenbarung der Erfindung

Der Erfindung liegt die Aufgabe zugrunde eine verbesserte Vorrichtung zur extrakorporalen Temperierung von Patienten zu bieten. Insbesondere sollte die Hygiene verbessert werden oder es sollte die übertragbare Wärmeleistung verbessert werden oder der Aufbau sollte vereinfacht sein oder einfacher zu reinigen sein oder anderweitig die Einhaltung von Hygienestandards erleichtern.

Gemäß einem Aspekt der Erfindung ist eine Vorrichtung zur extrakorporalen Temperierung von Patienten mit einer Übertragung von Wärme zwischen einem Fluid und einem Übertragungsmedium gemäß Anspruch 1 vorgesehen. Eine Vorrichtung umfasst einen Primärkreis mit einem Primärkörper zur Aufnahme des Fluids und einen Sekundärkreis mit einem Sekundärkörper, welcher wiederum Anschlüsse zur Zu- und Abführung des Übertragungsmediums umfasst, wobei der Sekundärkörper an dem Primärkörper befestigbar ist und Düsen zum Ausbilden einer Prallströmung aufweist, welche bei an dem Primärkörper befestigtem Sekundärkörper in Richtung des Primärkörpers gerichtet sind.

Ein Sekundärkörper ist insbesondere angepasst zur Verwendung in einer der hierin beschriebenen typischen Vorrichtungen mit Primärkörper, bspw. zur Befestigung an einem der hierin beschriebenen Primärkörper zur Ausbildung eines Wärmetauschers.

Weiter ist ein Verfahren zur extrakorporalen Temperierung von Patienten beschrieben, mit einer Übertragung von Wärme zwischen einem Fluid und einem Übertragungsmedium.

Als Prallströmung wird hierin eine Strömung bezeichnet, die typischerweise entsteht, wenn eine Fluid durch eine Düse, wie beispielsweise einen Schlitz oder eine Bohrung austritt und auf ein Strömungshindernis treffen. Im Freistrahlgebiet des Prallstrahls findet eine Aufweitung des Prallstrahls statt, da dieser von dem stromabwärts bereits vorhandenem Fluid beeinflusst wird. Der Potenzialkern des Prallstrahls wird dabei zunehmend kleiner mit dem Abstand zur Düse. Im Stauströmungsgebiet wird der Strahl durch ein Strömungshindernis, wie beispielsweise eine Fläche, eine Platte oder eine Wand beeinflusst und durch das Strömungshindernis zur Seite abgelenkt. Es können typischerweise turbulente oder laminare Strömungen realisiert werden. Der Wärmeübergang findet zu einem wesentlichen Teil in der Prallstrahlmitte im Stauströmungsgebiet statt und nimmt zur Seite hin ab. In einer typischen Ausführungsform weist die Vorrichtung einen Primärkörper auf. Der Primärkörper ist typischerweise Teil eines Primärkreises, in welchem ein Fluid aufnehmbar ist. Typischerweise ist das Fluid eine Flüssigkeit oder ein Gas oder ein Gas-Flüssigkeitsgemisch, das zur Speicherung und Übertragung von Wärme geeignet ist, beispielsweise Wasser, Pflanzenöl, Ammoniak, Kohlenstoffdioxid, Kohlenwasserstoffe, anorganische Kältemittel oder Luft. Unter dem Begriff Wärme oder Übertragung von Wärme wird hierin auch Kälte oder die Übertragung von Kälte verstanden. Die Begriffe "Wärme" und "Übertragung von Wärme werden hierin in einem physikalischen Sinn verstanden.

Typischerweise weist der Primärkörper ein Grenzelement zur Übertragung von Wärme auf das Übertragungsmedium auf. Typischerweise ist das Übertragungsmedium eine Flüssigkeit. Bei typischen Ausführungsformen umfasst das Übertragungsmedium mindestens 90 Gew.-% oder mindestens 95 Gew.-% Wasser, insbesondere Wasser für Injektionszwecke (WFI). In einer typischen Ausführungsform ist das Übertragungsmedium ein physiologisches oder isotonisches Medium. Typischerweise ist das Übertragungsmedium steril oder weitgehend keimfrei, beispielsweise weniger als 1000 oder maximal 100 koloniebildende, aerobe Keime pro Liter. Physiologische oder isotonische Medien sind beispielsweise eine 0,9%ige Lösung mit Kochsalz (Natriumchlorid, NaCl) oder eine Ringer-Lösung.

Typischerweise weist der Primärkörper ein Grenzelement auf, das eine Platte umfasst oder als Platte ausgebildet ist. In einer typischen Ausführungsform weist die Platte eine ebene oder eine glatte Oberfläche auf. Typischerweise kann die Platte strukturiert sein. Weitere typische Ausführungsformen umfassen gekrümmte Platten oder allgemein gekrümmte Oberflächen, welche das Grenzelement bilden.

Typischerweise ist die Platte aus einem Metall oder einer Metalllegierung, beispielsweise einer Titanlegierung oder einer Legierung mit Eisen. Bei weiteren Ausführungsformen umfasst die Platte einen Keramikwerkstoff. In einer typischen Ausführungsform ist die Platte aus einem Material gefertigt, das sich zur Wärmeübertragung eignet. Typischerweise ist die Platte an einer Seite des Primärkörpers angeordnet. Typischerweise ist die Platte von außen am Primärkörper zugänglich. Die Platte bildet bei typischen Ausführungsformen eine Außenseite des Primärkörpers. Bei weiteren Ausführungsformen kann eine abnehmbare Schutzplatte vor der Platte ausgebildet sein. Weitere Ausführungsformen umfassen Primärkörper, welche an mehreren Seiten Grenzelemente, beispielsweise Platten oder abgerundete Oberflächen aufweisen.

In einer typischen Ausführungsform weist der Sekundärkörper eine Dichtung auf, welche einen an dem Primärkörper befestigten Sekundärkörper gegen das Grenzelement abdichtet. Typischerweise weist der Sekundärkörper zumindest eine der folgenden Dichtungen auf: eine Labyrinthdichtung, eine Spaltdichtung, eine Dichtung aus einem dichtenden Material wie beispielsweise Kunststoff, Metall oder organische und/oder anorganische Materialien, eine Flachdichtung, oder eine stoffschlüssige Dichtung. Typischerweise ist die Dichtung am Sekundärkörper ausgeführt. In einer typischen Ausführungsform ist die Dichtung am Primärkörper oder als eigenes Element ausgeführt.

In einer typischen Ausführungsform ist der Sekundärkörper vom Primärkörper trennbar oder am Primärkörper insbesondere wiederholt befestigbar. Typischerweise ist der Sekundärkörper beschädigungsfrei vom Primärkörper trennbar. In einer typischen Ausführungsform ist der Sekundärkörper wiederholbar trennbar vom Primärkörper. Typischerweise sind erste und zweite Sekundärkörper trennbar oder beschädigungsfrei trennbar vom Primärkörper, wobei erste und zweite Sekundärkörper unterschiedlich voneinander oder identisch aufgebaut sein können. Ein vom Primärkörper trennbar ausgeführter Sekundärkörper kann den Vorteil bieten, dass eine Vorrichtung oder ein Sekundärkörper zur Verwendung in einer Vorrichtung keimfrei ist.

Typischerweise ist der Sekundärkörper zur einmaligen Nutzung ausgebildet. In einer typischen Ausführungsform ist der Sekundärkörper zur mehrfachen Nutzung ausgebildet. Bei einer einmaligen Nutzung ist der Sekundärkörper beispielsweise als Wegwerfartikel oder Einmalartikel ausgebildet. Dadurch kann eine zuverlässige Einhaltung von Hygienestandards erreicht werden. Bei typischen Ausführungsformen ist der Sekundärkörper autoklavierbar aufgebaut. Auf diese Weise lässt dich der Sekundärkörper mehrmals verwenden, wodurch Abfall eingespart werden kann.

In einer typischen Ausführungsform ist der Sekundärkörper im Wesentlichen aus Kunststoff gefertigt. Typischerweise ist der Sekundärkörper zumindest im Wesentlichen aus Kunststoff. Typischerweise ist der Sekundärkörper zu mindestens 90%, oder zu mindestens 80%, oder zu mindestens 70% aus Kunststoff. Die Angaben beziehen sich dabei auf Gewichts-%. Typischerweise ist der Sekundärkörper zu weniger als 100% aus Kunststoff, oder zu weniger als 50% aus Kunststoff. In einer typischen Ausführungsform ist der Sekundärkörper zumindest im Wesentlichen zu 100% aus Metall. Typischerweise ist der Sekundärkörper zu mindestens 90%, oder zu mindestens 80%, oder zu mindestens 70% aus Metall. Typischerweise ist der Sekundärkörper zu weniger als 100% aus Metall, oder zu weniger als 50% aus Metall.

In einer typischen Ausführungsform weist die Vorrichtung ein Verbindungselement zur lösbaren Befestigung des Sekundärkörpers an dem Primärkörper auf. Typische Verbindungselemente können ein an dem Primärkörper und dem Sekundärkörper ausgebildeter Bajonettverschluss oder Klammern oder Gewinde sein. Auf diese Weise ist eine beschädigungsfreie oder schnelle Montage oder Demontage des Sekundärkörpers möglich.

Typischerweise weist das Grenzelement einen Wärmetauscher auf. In einer typischen Ausführungsform ist das Grenzelement zweiteilig ausgeführt, wobei ein erstes Teilelement des Grenzelements eine Platte ist und ein zweites Teilelement Strukturen aufweist, die einen Durchfluss des Fluids des Primärkreises erlauben. Typischerweise sind das erste und das zweite Teilelement des Grenzelements stoffschlüssig oder formschlüssig verbunden. In einer typischen Ausführungsform ist das Grenzelement einteilig ausgeführt, wobei das Grenzelement Strukturen aufweist, die den Durchfluss des Fluids erlauben. Das Grenzelement ist typischerweise zur Übertragung von Wärme geeignet. In einer typischen Ausführungsform umfasst das Fluid des Primärkreises Wasser (H₂O), ein pflanzliches oder mineralisches Öl, Ammoniak, Kohlenstoffdioxid, Kohlenwasserstoffe, oder anorganische Kältemittel.

In einer typischen Ausführungsform ist ein Sekundärkörper zur Verwendung in einer der hierin beschriebenen typischen Vorrichtungen zur extrakorporalen Temperierung von Patienten vorgesehen.

Typischerweise umfasst der Sekundärkörper eine erste Kammer mit einem ersten Anschluss zur Zufuhr des Übertragungsmediums in die erste Kammer und eine zweite Kammer mit einem zweiten Anschluss zur Abfuhr des Übertragungsmediums aus der zweiten Kammer, wobei die zweite Kammer eine offene Seite aufweist. Typische Sekundärkörper weisen mindestens eine offene Seite auf. Typischerweise sind die Düsen des Sekundärkörpers in Richtung der offenen Seite ausgerichtet.

In einer typischen Ausführungsform sind die erste Kammer des Sekundärkörpers und die zweite Kammer des Sekundärkörpers durch mindestens eine oder durch eine Mehrzahl auf die offene Seite der zweiten Kammer gerichtete Düse verbunden, um eine Strömung des Übertragungsmediums auf die offene Seite zu richten.

Typischerweise weist mindestens eine der Düsen einen runden Querschnitt auf. Bei typischen Ausführungsformen weist mindestens eine der Düsen einen eckigen oder einen länglichen Querschnitt auf. Bei einer typischen Ausführungsform ist die mindestens eine Düse als Venturi-Düse ausgeführt. Typischerweise sind die erste und die zweite Kammer des Sekundärkörpers über mindestens zwei, mindestens 10, oder mindestens 100 Düsen miteinander verbunden. Typischerweise sind die erste und die zweite Kammer des Sekundärkörpers mit weniger als 1000 Düsen, weniger als 500 Düsen miteinander verbunden.

Typischerweise sind zumindest einige oder alle der Düsen, die die erste Kammer und die zweite Kammer des Sekundärkörpers verbinden, als Düsenfeld angeordnet. Bei typischen Ausführungsformen sind die Düsen randomisiert angeordnet.

In typischen Ausführungsformen umfasst die erste Kammer des Sekundärkörpers ein Sieb. Typischerweise ist das Siebe vor, also stromaufwärts von zumindest einer, oder vor allen Düsen angeordnet. Typischerweise ist das Sieb so angeordnet, dass ein Fluid zuerst durch das Sieb strömt, bevor es zu einer Düse gelangt. Typischerweise ist das Sieb so angeordnet, dass ein Verstopfen der Düsen verhindert werden kann. Das Sieb kann den Vorteil bieten, dass die Betriebssicherheit erhöht wird.

Bei typischen Ausführungsformen weist der Sekundärkreis mindestens eine Vorrichtung zur Abscheidung von Luftblasen auf. Während einer Befüllung des Sekundärkreises mit dem Übertragungsmedium kann verbleibende Luft aus dem Sekundärkreis auf diese Weise passiv entfernt werden.

Bei typischen Ausführungsformen umfasst ein Verfahren zur extrakorporalen Temperierung von Patienten mit einem Primärkreis mit einem Primärkörper zur Aufnahme des Fluids und einem Sekundärkreis mit einem Sekundärkörper, welcher Anschlüsse zur Zu- und Abfuhr des Übertragungsmediums umfasst, folgende Schritte: Befestigen eines ersten Sekundärkörpers an dem Primärkörper, Temperieren eines ersten Übertragungsmediums in dem ersten Sekundärkörper mittels des durch den Primärkörper geführten Fluids, Trennen des ersten Sekundärkörpers von dem Primärkörper mit gegebenenfalls Entfernen des ersten Übertragungsmediums, Reinigen des Primärkörpers, insbesondere Reinigen zumindest des Bereichs des Grenzelements, welches mit dem ersten Übertragungsmedium in Berührung kam, Befestigen eines zweiten Sekundärkörpers an dem Primärkörper, Temperieren eines zweiten Übertragungsmediums in dem zweiten Sekundärkörper mittels des durch den Primärkörper geführten Fluids, Trennen des zweiten Sekundärkörpers vom Primärkörper.

Typischerweise wird das zweite Übertragungsmedium vor oder nach dem Trennen des zweiten Sekundärkörpers entfernt. Bei typischen Ausführungsformen erfolgt nach dem Trennen ein Reinigen des Primärkörpers, insbesondere Reinigen zumindest des Bereichs des Grenzelements, welches mit dem zweiten Übertragungsmedium in Berührung kam: Anschließend kann das Verfahren mit einem dritten und analog weiteren Sekundärkörpern und einem dritten und analog weiteren Übertragungsmedien fortgesetzt werden. Typischerweise erfolgt nach jeder Temperierung eines Patienten ein Wechsel des Sekundärkörpers oder ein Autoklavieren des Sekundärkörpers und ein Entfernen, also typischerweise Entsorgen, des Übertragungsmediums. Auf diese Weise können Hygienestandards leicht eingehalten werden. Typischerweise basiert die Übertragung von Wärme zwischen dem Fluid und dem Übertragungsmedium auf dem Prinzip der Prallströmung.

Das Grenzelement weist typischerweise eine Fläche von mindestens 0,01 m*m, mindestens 0,02 m*m, mindestens 0,03 m*m, mindestens 0,04 m*m oder mindestens 0,2 m*m auf. Das Grenzelement weist eine Fläche von weniger als 1 m*m, oder weniger als 0,5 m*m oder weniger als 0,1 m*m. Auf diese Weise lassen sich mit einer kompakten Vorrichtung zuverlässig ausreichende Wärmeleistungen der Vorrichtung zur Temperierung eines Patienten erzielen. Bei typischen Ausführungsformen kann das Grenzelement eine strukturierte Oberfläche aufweisen, wie beispielsweise mit Rippen oder Mikrostrukturen.

### Kurze Beschreibung der Zeichnungen

Nachfolgend werden Ausführungsbeispiele der Erfindung anhand von Zeichnungen näher erläutert. Darin zeigen:
Fig. 1 einen schematischen Aufbau der Vorrichtung mit einem Primärkörper und einem Sekundärkörper;
Fig. 2 einen schematischen Schnitt durch einen Sekundärkörper mit einer ersten Kammer und einer zweiten Kammer, wobei der Sekundärkörper trennbar mit einem Primärkörper verbunden ist;
Fig. 3 ein Verfahren in einer typischen Ausführungsform, wobei das Verfahren keine Ausführungsform der Erfindung ist.

### Beschreibung der in den Figuren gezeigten Ausführungsbeispiele

Nachfolgend werden typische Ausführungsbeispiele der Erfindung beschrieben, wobei für gleiche oder ähnliche Teile gleiche Bezugszeichen verwendet werden und nicht mit jeder Figur nochmals erläutert werden. Die Erfindung ist nicht auf die nachfolgend beschriebenen typischen Ausführungsformen beschränkt.

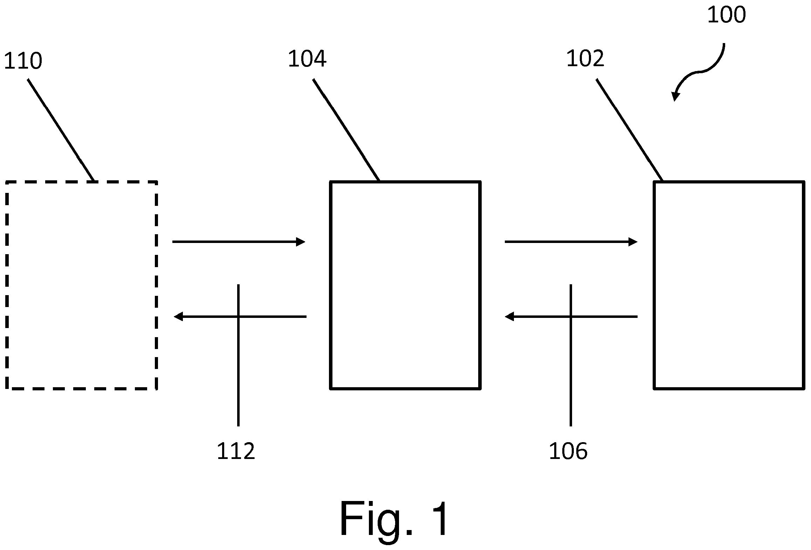

In der Fig. 1 ist schematisch eine typische Ausführungsform der Vorrichtung 100 gezeigt. Typischerweise umfasst die Vorrichtung 100 einen zum Wärmetransport vorgesehenen Primärkreis mit einem Primärkörper 102 und einen zum Wärmetransport vorgesehenen Sekundärkreis mit einem Sekundärkörper 104.

Der Primärkreis mit dem Primärkörper 102 überträgt Wärme 106 zu dem Sekundärkörper 104. Mit dieser Formulierung ist wie allgemein in dieser Anmeldung auch ein Transport von Wärme in umgekehrter Richtung, d.h. eine Kühlung, umfasst. Die Wärmeübertragung 106 wird typischerweise über eine oder eine Mehrzahl an Düsen realisiert, die eine Prallströmung ausbilden. Der Sekundärkreis überträgt Wärme 112 von dem Sekundärkörper 104 mittels eines Übertragungsmediums, im dargestellten Beispiel mittels einer 0,9%-NaCl-Lösung (isotonische Lösung) zu einem Oxygenator 110, in welchem die Wärme auf das Blut eines Patienten übertragen werden kann. Wiederum ist wie an jeder anderen Stelle dieser Anmeldung mit dem Begriff "Wärme übertragen" auch ein Entzug von Wärme, also ein Kühlen, umfasst. Auf diese Weise kann ein Patient, dessen Blutkreislauf zumindest teilweise durch den Oxygenator 110 geführt wird, temperiert, d.h. gekühlt oder erwärmt, werden.

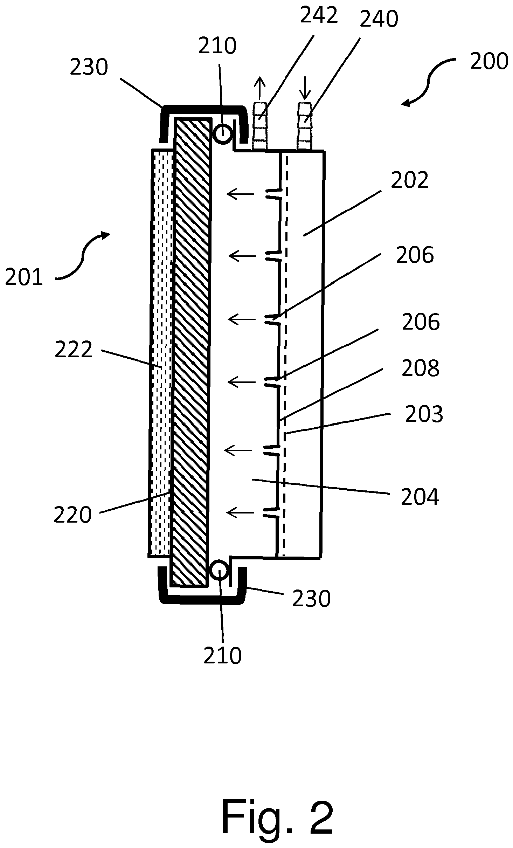

In der Fig. 2 ist ein schematischer Schnitt durch einen typischen Sekundärkörper 200 in einer Seitenansicht gezeigt. Der Sekundärkörper 200 ist mit einem Primärkörper 201 trennbar verbunden.

Der Sekundärkörper 200 umfasst eine erste Kammer 202 mit einem Anschluss 240 für die Zufuhr eines Übertragungsmediums und eine zweite Kammer 204 mit einem Anschluss 242 für die Abfuhr des Übertragungsmediums. Die erste Kammer 202 des Sekundärkörpers 200 umfasst ein Sieb 203.

Die erste Kammer 202 ist mit der zweiten Kammer 204 durch Düsen 206, die in einem Düsenfeld 208 organisiert angeordnet sind, verbunden. Das Sieb 203 ist so angeordnet, dass ein Fluid zuerst auf das Sieb 203 trifft, bevor es durch die Düsen 206 in die zweite Kammer 204 gelangt, das Sieb 203 ist also stromaufwärts der Düsen 206 angeordnet. Die zweite Kammer 204 weist eine offene Seite auf. Die Düsen 206 des Düsenfelds 208 sind auf die offene Seite der zweiten Kammer 204 gerichtet. Typischerweise bilden die eine oder die Mehrzahl an Düsen jeweils einen Prallstrahl aus.

Die offene Seite der zweiten Kammer 204 weist eine um die offene Seite umlaufende Dichtung 210 auf. Die Dichtung 210 dichtet die offene Seite der zweiten Kammer 204 gegen ein Grenzelement 220 des Primärkörpers 201 ab. Das Grenzelement 220 umfasst in der in der Fig. 2 gezeigten beispielhaften Ausführungsform eine Platte bzw. ist bei dem dargestellten typischen Ausführungsbeispiel der Fig. 2 als Platte ausgebildet. Die Platte des Grenzelements 220 ist mit einer Wärmetauscherstruktur 222 des Primärkörpers 201 verbunden. In typischen Ausführungsformen trifft der eine oder die Mehrzahl an Prallstrahlen auf das Grenzelement oder eine Platte des Grenzelements und bilden eine Prallströmung aus.

Typischerweise ist die Verbindung zwischen der Wärmetauscherstruktur und der Platte stoffschlüssig oder formschlüssig. Bei typischen Ausführungsformen ist die Wärmetauscherstruktur in der Platte integriert.

Die Wärmetauscherstruktur 222 weist in der beispielhaften Ausführungsform der Fig. 2 Kanäle auf, die von einem Fluid durchströmt werden.

Das Fluid wird typischerweise mittels eines Wärmetauschers des Primärkreises temperiert.

In der beispielhaften Ausführungsform der Fig. 2 ist das Grenzelement 220 des Primärkörpers 201 über eine trennbare Verbindung 230 in Form von Spannklammern mit einer Struktur, beispielsweise einem Rand, der zweiten Kammer 204 des Sekundärkörpers 200 verbunden. An dem Rand ist bei typischen Ausführungsformen die Dichtung 210 angeordnet. Auf diese Weise wird eine einfache und zuverlässige Abdichtung erreicht.

Typischerweise ist die trennbare Verbindung eine formschlüssige Verbindung. Eine typische trennbare Verbindung kann beispielsweise als Bajonettverschluss oder als Spannklammern ausgeführt sein. In einer typischen Ausführungsform ist der Sekundärkörper rund ausgeführt. Typischerweise kann der Sekundärköper auch eckig ausgeführt sein.

Typischerweise dichtet die Dichtung den Sekundärkörper gegen das Grenzelement des Primärkörpers ab. In einer typischen Ausführungsform dichtet die Dichtung eine offene Seite des Sekundärkörpers seitlich gegen ein Grenzelement des Primärkörpers oder gegen den Primärkörper ab. Typischerweise ist die Dichtung einteilig ausgeführt. In einer typischen Ausführungsform ist die Dichtung mit mindestens zwei Teilen ausgeführt, die eine offene Seite des Sekundärkörpers gegen einen unkontrollierten Austritt von Übertragungsmedium abdichten.

In einer typischen Ausführungsform weist die offene Seite des Sekundärkörpers mehrere Öffnungen auf. Typischerweise weist die offene Seite des Sekundärkörpers eine oder mehr Öffnungen auf.

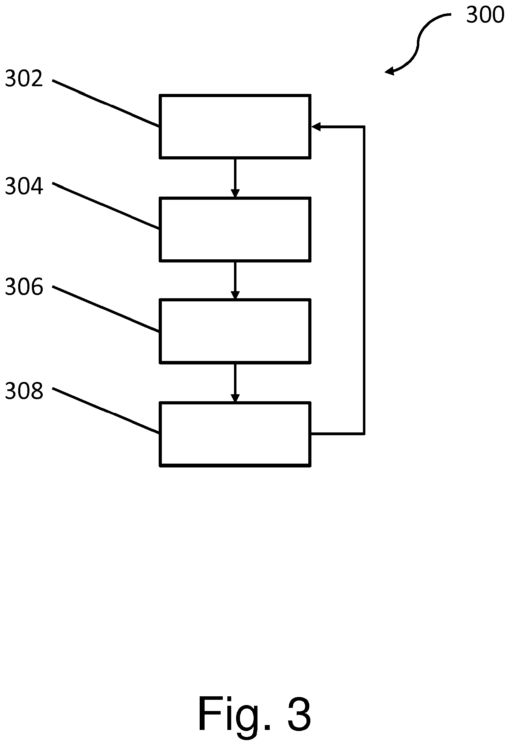

In der Fig. 3 ist schematisch ein typisches Verfahren 300 zur extrakorporalen Temperierung von Patienten, mit einer Übertragung von Wärme mittels eines Fluids und eines Übertragungsmediums gezeigt.

Im beispielhaften Verfahren 300, wie in Fig. 3 gezeigt, wird in 302 ein erster Sekundärkörper mit einem Primärkörper trennbar verbunden. Die trennbare Verbindung ist typischerweise beschädigungsfrei trennbar. Typischerweise ist die trennbare Verbindung gegen ein unkontrolliertes Austreten von Übertragungsmedium abgedichtet.

In 304 wird ein erstes Übertragungsmedium in dem ersten Sekundärkörper mittels des durch den Primärkörper geführten Fluids temperiert.

In 306 wird der erste Sekundärkörper von dem Primärkörper beschädigungsfrei getrennt. In 308 wird das Grenzelement des Primärkörpers oder der Primärkörper gereinigt. Typischerweise umfasst eine Reinigung eine Sterilisierung des Primärkörpers oder zumindest von Teilen des Primärkörpers, wie beispielsweise des Grenzelements.

Das Verfahren springt anschließend für den nächsten Patienten zu 302 zurück, wobei dann im zweiten Durchlauf ein zweiter Sekundärkörper und ein zweites Übertragungsmedium zum Einsatz kommen, so dass keine im ersten Durchlauf in das erste Übertragungsmedium eingetragenen Keime zu einer Verunreinigung führen. Ein dritter und weitere Durchläufe des Verfahrens können zur Temperierung von weiteren Patienten erfolgen.

Die Wiederholung kann auch mit einem sterilisierten Sekundärkörper erfolgen

## Patentansprüche

1. Vorrichtung zur extrakorporalen Temperierung von Patienten, mit einer Übertragung von Wärme zwischen einem Fluid und einem Übertragungsmedium, mit
- einem Primärkreis mit einem Primärkörper (102, 201) zur Aufnahme des Fluides, und
- einem Sekundärkreis mit einem Sekundärkörper (104, 200), welcher Anschlüsse (240, 242) zur Zu- und Abführung des Übertragungsmediums umfasst,
wobei der Sekundärkörper (104, 200) an dem Primärkörper (102, 201) befestigbar ist,
wobei der Sekundärkörper (104, 200) zumindest im Wesentlichen aus Kunststoff gefertigt ist, und
wobei der Primärkörper (102, 201) ein Grenzelement (220) zur Übertragung von Wärme auf das Übertragungsmedium umfasst, wobei das Grenzelement (220) eine Fläche von weniger als 1m*m aufweist, **dadurch gekennzeichnet,**
**dass** der Sekundärkörper (104, 200) mindestens eine Düse (206) zum Ausbilden einer Prallströmung aufweist, welche bei an dem Primärkörper (102, 201) befestigtem Sekundärkörper (104, 200) in Richtung des Primärkörpers (102, 201) gerichtet ist.

2. Vorrichtung nach Anspruch 1, wobei das Grenzelement (220) eine Platte umfasst.

3. Vorrichtung nach dem vorhergehenden Anspruch, wobei der Sekundärkörper (104, 200) eine eine offene Seite des Sekundärkörpers umschließende Dichtung (210) aufweist, welche einen an dem Primärkörper (102, 201) befestigten Sekundärkörper (104, 200) gegen das Grenzelement abdichtet.

4. Vorrichtung nach einem der vorhergehenden Ansprüche, wobei der Sekundärkörper (104, 200) vom Primärkörper (102, 201) trennbar ist.

5. Vorrichtung nach einem der vorhergehenden Ansprüche, wobei der Sekundärkörper (104, 200) zur einmaligen Nutzung ausgebildet ist.

6. Vorrichtung nach einem der vorhergehenden Ansprüche, mit einem Verbindungselement (230) zur lösbaren Befestigung des Sekundärkörpers (104, 200) an dem Primärkörper (102, 201).

7. Vorrichtung nach einem der vorhergehenden Ansprüche, wobei das Grenzelement (220) einen Wärmetauscher aufweist.

## Claims

1. Device for extracorporeal temperature control of patients, with a transfer of heat between a fluid and a transfer medium, comprising
- a primary circuit having a primary body (102, 201) for receiving the fluid, and
- a secondary circuit having a secondary body (104, 200) which comprises connections (240, 242) for supplying and removing the transfer medium,
the secondary body (104, 200) being fastenable to the primary body (102, 201),
the secondary body (104, 200) being made at least substantially of plastics material, and
the primary body (102, 201) comprising a boundary element (220) for transferring heat to the transfer medium, the boundary element (220) having an area of less than 1m*m, **characterised in that**
the secondary body (104, 200) has at least one nozzle (206) for forming an impact flow which, when the secondary body (104, 200) is fastened to the primary body (102, 201), is directed in the direction of the primary body (102, 201).

2. Device according to claim 1, wherein the boundary element (220) comprises a plate.

3. Device according to the preceding claim, wherein the secondary body (104, 200) has a seal (210) which encloses an open side of the secondary body and which seals a secondary body (104, 200) fastened to the primary body (102, 201) with respect to the boundary element.

4. Device according to any of the preceding claims, wherein the secondary body (104, 200) is separable from the primary body (102, 201).

5. Device according to any of the preceding claims, wherein the secondary body (104, 200) is designed for single use.

6. Device according to any of the preceding claims, comprising a connecting element (230) for releasably fastening the secondary body (104, 200) to the primary body (102, 201).

7. Device according to any of the preceding claims, wherein the boundary element (220) has a heat exchanger.

## Revendications

1. Dispositif permettant la régulation de la température extracorporelle de patients par un transfert de chaleur entre un fluide et un milieu de transfert, comportant
- un circuit primaire comportant un corps primaire (102, 201) destiné à recevoir le fluide, et
- un circuit secondaire comportant un corps secondaire (104, 200), qui comprend des raccords (240, 242) pour l'amenée et l'évacuation du milieu de transfert,
dans lequel le corps secondaire (104, 200) peut être fixé au corps primaire (102, 201),
dans lequel le corps secondaire (104, 200) est au moins sensiblement fabriqué en matière plastique, et
dans lequel le corps primaire (102, 201) comprend un élément de limite (220) permettant de transférer de la chaleur au milieu de transfert, dans lequel l'élément de limite (220) présente une surface inférieure à 1 m*m, **caractérisé en ce**
**que** le corps secondaire (104, 200) présente au moins une buse (206) destinée à former un flux d'impact qui, lorsque le corps secondaire (104, 200) est fixé au corps primaire (102, 201), est dirigé en direction du corps primaire (102, 201).

2. Dispositif selon la revendication 1, dans lequel l'élément de limite (220) comprend une plaque.

3. Dispositif selon la revendication précédente, dans lequel le corps secondaire (104, 200) présente un joint d'étanchéité (210) entourant un côté ouvert du corps secondaire, lequel joint d'étanchéité assure l'étanchéité d'un corps secondaire (104, 200) fixé au corps primaire (102, 201) contre l'élément de limite.

4. Dispositif selon l'une des revendications précédentes, dans lequel le corps secondaire (104, 200) peut être séparé du corps primaire (102, 201).

5. Dispositif selon l'une des revendications précédentes, dans lequel le corps secondaire (104, 200) est conçu pour être utilisé une seule fois.

6. Dispositif selon l'une des revendications précédentes, comportant un élément de liaison (230) permettant de fixer de manière amovible le corps secondaire (104, 200) au corps primaire (102, 201).

7. Dispositif selon l'une des revendications précédentes, dans lequel l'élément de limite (220) présente un échangeur de chaleur.
